# EUROPEAN PATENT APPLICATION

(11) **EP 1 826 264 A1**
(43) Date of publication of application: **29.08.2007**
(21) Application number: 06003847.8
(22) Date of filing: 24.02.2006
(51) Int. Cl.: C12N 7/00, C07K 14/065, A61K 35/76, A61K 39/275

(54) **Primate model for orthopox virus infections**

(71) Applicant: Deutsches Primatenzentrum GmbH, 37077 Goettingen (DE)
(72) Inventor: Kaup, Franz-Josef, Prof. Dr., 31535 Neustadt (DE); Mätz-Rensing, Kerstin, Dr., 37191 Katlenburg-Lindau (DE); Stahl-Hennig, Christiane, Dr., 37083 Göttingen (DE); Pauli, Georg, Prof., 10785 Berlin (DE); Ellerbrok, Heinz, Dr., 13353 Berlin (DE)
(74) Representative: Reinhard - Skuhra - Weise & Partner

(57) **Abstract**

The present invention is directed to an animal model for predicting the course of orthopox virus infections or for evaluating the efficacy of anti-viral therapeutics or anti-orthopox virus vaccines. The present invention is further directed to a method of evaluating the efficacy of an anti-viral therapeutic or an anti-orthopox virus vaccine and to a specific Callithrixpox virus strain for use in this method. Furthermore, the present invention pertains to a pharmaceutical composition containing such an anti-viral therapeutic or an anti-orthopox virus vaccine.

### Annex to the application documents - subsequently filed sequences listing

SEQUENCE LISTING

<110>  Deutsches Primatenzentrum GmbH

<120>  Primate model for orthopox virus infections

<130>  P20053

<140>  EP 06003847.8
<141>  2006-02-24

<160>  2

<170>  PatentIn version 3.3

<210>  1
<211>  21
<212>  DNA
<213>  Artificial

<220>
<223>  Forward primer

<400>  1
atgccgcaac aactatctcc t                                                21


<210>  2
<211>  22
<212>  DNA
<213>  Artificial

<220>
<223>  Reverse primer

<400>  2
ctagttttgt ttttctcgcg aa                                               22

## Description

The present invention is directed to an animal model for predicting the course of orthopox virus infections or for evaluating the efficacy of anti-viral therapeutics or anti-orthopox virus vaccines. The present invention is further directed to a method of evaluating the efficacy of an anti-viral therapeutic or an anti-orthopox virus vaccine and to a specific Callithrixpox virus strain for use in this method. Furthermore, the present invention pertains to a pharmaceutical composition containing such an anti-viral therapeutic or an anti-orthopox virus vaccine.

Although pox virus infections are not common, spontaneous outbreaks of corresponding diseases occur among non-human primate populations. In particular, four members of the pox virus family belonging to Orthopox virus, Yatapox virus and Molluscipox virus genera cause disease in nonhuman primates. The most important one is the infection with monkeypox virus, an orthopox virus closely related to smallpox virus of man.¹

Old World monkey species seem to have a higher risk of getting the infection than New World monkeys. Nevertheless, outbreaks were also observed among cotton-top tamarins and squirrel monkeys.^{17,18} The infection leads to a generalized disease characterized by circular proliferating lesions of the skin that undergo vesiculation and ulceration or developed into pustules that crust over and drop off, leaving small scars. Other poxviruses belonging to the molluscum contagiousum group were identified as causative agent of subcutaneous tumor-like lesions. Natural infections with this Yabapox virus have occurred in rhesus monkeys and baboons.¹³ The disease is characterized by rapidly growing subcutaneous nodules on head and limbs.⁵ The infection seems not to occur in New World monkeys. This also applies for Tanapox viruses which are unrelated to smallpox virus and capable of inducing a contagious skin disease in macaques and humans. The infection leads to multiple crusted macules on face and arms, which heal in three to four weeks and resemble monkeypox arrested at the papular stage. Pathologically, the lesions are characterized by epidermal hyperplasia. Swollen epithelial cells contain eosinophilic cytoplasmic inclusion bodies. The disease is not transmissible to cebids and callithrichids.¹⁵

Another poxvirus, serologically closely related to Tanapox virus has been isolated from a spontaneous outbreak in *Callithrix jacchus.*¹⁴ The single epizootic outbreak began three weeks after importation and lasted for six months. Thirty six percent of 80 marmosets developed cutaneous erythematous papules, particularly on the tails, scrotum and abdomen, which progressed within ten days to elevated plaques with scab formation or to small hemorrhagic ulcers. Coincidentally, multiple plaques with dark umbilicated centers were seen on the palms and soles. Pox-like intracytoplasmic inclusions were evident and viral particles characteristic for the genus were found by ultrastructural examination. The clinical disease endured over four to six weeks. Occurring deaths were attributed to intercurrent diseases. In the literature no data about cow-pox-infection among nonhuman primates were available. In this report a detailed clinicopathological description of an uncommon orthopox virus epizootic outbreak among different New World monkey species is given.

In Jahrling et al., 2004, PNAS 101, 15196-15200, it is disclosed to expose Cynomolgus macaques to several variola strains through aerosol and/or i.v. routes. Two strains, Harper and India 7124, produced uniform acute lethality when inoculated i.v. in high doses (10(9) plaque-forming units). Lower doses resulted in less fulminant, systemic disease and lower mortality. Animals that died had profound leukocytosis, thrombocytopenia, and elevated serum creatinine levels.

This animal model, however, uses highly infectious and harmful variola strains and the safety risks of this approach only allows to perform this model under S4 safety conditions. Furthermore, the survival time of the macaques varied considerably from between 3 to 10 days and thus, this model is hardly suitable for testing the efficacy of a new smallpox vaccine. In this connection it is noted that an animal model for use as a test system for smallpox vaccines should allow an evaluation of its efficacy reliably for more than at least 4 days, since it is known for conventional smallpox vaccines that a vaccination may also be successful at 4 days post infection.

Hooper et al., "Smallpox DNA vaccine protects nonhuman primates against lethal monkeypox", J Virol. 2004 May; 78(9):4433-43, describe that rhesus macaques vaccinated with a DNA vaccine consisting of four vaccinia virus genes (L1R, A27L, A33R, and B5R) were protected from severe disease after an otherwise lethal challenge with monkeypox virus. Animals vaccinated with a single gene (L1R) which encodes a target of neutralizing antibodies developed severe disease but survived. However, in this animal model, macaques have to be infected with a high dosage (2 x 10⁷ to 5 x 10⁸ pfu) in order to cause reliable conditions. Furthermore, the tests have to be performed still under safety level S3.

Smallpox virus (variola) poses a significant threat as an agent of bioterrorism. Furthermore, this situation is exacerbated by the fact that nowadays only people at high risk are vaccinated against smallpox (since the declaration in 1980 by the WHO that smallpox had been eradicated). In order to lower this risk, antiviral drugs and improved vaccines are urgently needed. In this connection, satisfactory demonstration of protective efficacy against authentic variola thus requires the development of an animal model in which orthopox viruses other than variola produce a disease course with features consistent with human smallpox.

Therefore, it is one problem underlying the present invention to provide an animal model for orthopox virus infections, in particular variola infections, which is closely simulating the natural course of a smallpox infection in human beings.

It is a further problem underlying the present invention to provide an animal model, which is involving a species specific infection in order to avoid any transmission to human beings and which can be analyzed under lower safety conditions than the animal models known up to now.

A still further problem is to provide an animal model which allows the determination of new vaccines or anti-viral agents protective efficacy against smallpox infections.

These problems are solved by the subject-matter of the independent claims. Preferred embodiments are set forth in the dependent claims.

The present invention provides an animal model, which can be performed at relatively low safety levels (S2), is cost effective and allows a reliable prediction of the efficacy of vaccines or anti-viral therapeutics designed against orthopox virus infections, in particular smallpox infections in humans.

The scientific background of this invention was an atypical epizootic infection which was observed in a colony of 80 New World monkeys composed of different species, including a group of marmosets and *Saguinus* species. During summer and autumn 2002, thirty animals died. Six animals were analysed at the German Primate Center and the Robert Koch-Institut in order to examine the cause of death and for complete pathohistological investigation. The investigated animals exhibited erosive-ulcerative lesions of the oral mucous membranes.

Later stages were characterised by skin lesions presenting as hemorrhagic lesions distributed randomly over the body, but primarily on face, scrotal regions, soles and palms. Electron microscopy revealed virus particles with orthopox-like morphology within intracytoplasmatic inclusions. DNA from various tissues was analysed immediately with a set of orthopox virus specific real-time PCR assays, with conventional PCR and DNA sequencing techniques. The presence of orthopox virus was confirmed, excluding at the same time variola virus. Virus could be detected in different organs and sequence data showed that all six animals were infected with the same virus. Cultivation experiments with Vero cells resulted in rapidly progressing cytopathic plaques confirming the presence of infectious virus in the tissue samples. Preliminary phylogenetic analyses of several genes revealed that the virus is distinct but closely related to cowpox viruses. The source of this poxvirus outbreak remains to be elucidated and the nature of the virus has to be precisely determined.

As mentioned above, pox virus infections are a serious concern to public health. The described outbreak of an orthopox virus infection in a group of different New World monkeys attracts attention to the possible risk of this infection for other species including man. A cowpox virus, member of the genus orthopox virus, was identified as causative agent for this fatal infection among marmosets. During this outbreak, all infected animals developed a severe systemic infection with most prominent lesions on the oral mucous membranes, which is in line with the strong epitheliotropism of the virus. The most advanced lesions consisted of multifocal epidermal and mucosal vesicle formation, necrosis and ulceration attendant upon intense inflammatory response. Interestingly, only single animals developed typical papular skin lesions. A classic pustule formation only was observed in one case, the other animals died before reaching this stage of illness. The detection of hemorrhagic papular skin diseases in nonhuman primates should direct the attention to a contagious, possibly zoonotic disease.

The diagnosis of pox virus infections is based on the demonstration of typical intracytoplasmatic inclusion bodies within the altered epidermis. These bodies suggest viral inclusions, but electron microscopy molecular diagnostics methods and viral isolation techniques have to be used to confirm a poxvirus infection. Phylogenetic analyses of the isolated poxvirus revealed closest homology to cowpox viruses. Cowpox virus is a rodent virus that may infect cats, cows and zoo animals and through contact to these, also humans. Cowpox virus infections are endemic among the cattle population, although clinical cases in the European cattle population are rare. Field and experimental studies have indicated that cowpox have a broad host range and a wildlife reservoir in rodents and foxes. Furthermore the virus is often isolated from domestic cats, which should be regarded as important vector in urban areas.^{2,6,16} Lesions in humans can often be found on the hands, forearms, faces and necks. Predisposition, such as immunosuppression, may lead to a more severe or fatal course of infection like in a case of a glucocorticoid treated asthma patient after contact with a cat.^{8,10}

A careful evaluation of the epidemiological situation of cowpox virus infections suggests that cowpox have a low virulence and contiguousness for humans.³ Thus, an animal model involving cowpox could involve less safety problems.

The case described herein demonstrates that New World Monkeys of different species are susceptible. All infected animals were in a good condition and no predisposing immunosuppressive co-factors were known. The isolated virus has to be regarded as contagious for New World Monkeys.

There was no transmission to the animal owner, to animal keepers, clinical veterinarians or pathologists during this outbreak and the zoonotic potential of this pox virus is not established. The epidemiological situation in this epizootic is still under investigation. Three humans with a history of small pox vaccination had low titers of cross reacting antibodies against cowpox virus, an unvaccinated child was negative. There are no hints for a specific vector in this case, but it is assumed that rodents have brought the virus in the colony.

Antibodies against orthopox virus were demonstrated in 41 % of rodents caught in this area which were tested for antibodies using immunofluorescence.⁴

Rodents are thought to be a reservoir for cowpox virus and persistent infections as well as recombination events might occur.¹⁹ While cowpox viruses are known to infect various species, including cats, humans and other primates, they only cause localized self-limiting infections, and cowpox virus infections have not been observed in marmosets. However, a recombination event between different viral strains could have generated a virus with the observed new properties leading to fatal infection in marmosets with spreading of the virus to secondary organs with high viral titers displaying many symptoms also seen during smallpox infections in man. Furthermore, the virus seems to be transmitted from animal to animal, either by respiration or by skin contact establishing a chain of infection under natural conditions.

The present invention in particular is directed to the following aspects and embodiments.

In a first and most general aspect, the present invention is directed to an animal model for predicting the *in vivo* course of orthopox virus infections or for evaluating the efficacy of anti-viral therapeutics or anti-orthopox virus vaccines, comprising a New World monkey which has been infected with orthopox viruses.

As mentioned above, it turned out that New World monkeys are susceptible to infection with orthopox viruses, in particular cowpox viruses. Furthermore, New World monkeys like Callithrix are comparably cheap and may be held easily. Additionally, it turned out that the orthopox virus infections generated within theses monkeys showed a reliable course which may allow a prediction of the course of (smallpox) infections also in humans.

The New World monkeys or Platyrrhines are the four families of primates that are found in Central and South America, the Cebidae, Aotidae, Pitheciidae and Atelidae. All families differ from the Old World monkeys and apes in having long, often prehensile tails. Platyrrhine noses are flatter, with side facing nostrils, compared to the narrow noses and downward facing nostrils of Old World monkeys. Many are small, arboreal and nocturnal, so the knowledge of them is less comprehensive than that of the more easily observed Old World monkeys.

The marmosets are the genus *Callithrix* of New World monkeys and belong to the family of the Cerbidae. Most marmosets are about 20 cm in length. Relative to other monkeys, they show a number of characteristic features: they have claws rather than nails, and tactile hairs on their wrists.

It is noted that the orthopox viruses against which the anti-viral therapeutics or vaccines are designed and evaluated in the present animal model are not necessarily the same (and usually are not) like the orthopox viruses used for infecting the New World monkeys. As an example, it turned out that cowpox viruses of the genus *Callithrix* may be advantageously used for infecting the monkeys and provide a precise and reliable animal model for evaluating the efficacy of anti-viral therapeutics or vaccines against smallpox infections in humans.

Or in other words, the present animal model may be used in a screening method for determining, whether a specific anti-viral agent or vaccine may be effective against a disease in humans, like an infection of smallpox or monkeypox, or not.

In a preferred embodiment, the orthopox viruses used for infecting the New World monkey are selected from monkeypox or cowpox viruses. In particular the latter are preferred since they seem to be mostly harmless for humans thus allowing a comparably low safety standard to apply. Amongst the cowpox viruses, Callithrixpox viruses are in particular preferred.

Most preferably, this virus belongs to the strain deposited under the accession number ECACC 06013101.

The anti-viral therapeutics or anti-orthopox virus vaccines are intended for the prophylaxis or treatment of infections with monkeypox, cowpox or smallpox.

It is noted that the term "anti-viral therapeutic" as used herein includes all conceivable agents which may cause a reduction or even elimination of the pox virus load in a mammal, in particular in a human. The term is interchangeably used with an anti-viral agent or anti-viral drug for use against pox virus infections.

Preferred points of attack of such an anti-viral agent include attachment to a host cell, release of viral genes and possibly enzymes into the host cell, replication of viral components using host-cell machinery, assembly of viral components into complete viral particles and/or release of viral particles to infect new host cells. This can be done, for example, by blocking specific enzymes or transcription factors essential for viral synthesis, or by using nucleotide or nucleoside analogues etc.

The term is distinguished herein from the expression "vaccines". Vaccines mostly have an excellent protective effectiveness, but they are often of limited use in treating a patient who has already been infected. Antiviral drugs may preferably be used in treating an existing disease (infection with orthopox viruses) and for pre- and post-exposition prophylaxis.

Vaccines as used herein preferably are preferably live Orthopoxvirus vaccines, preferably based on or derived from vaccinia virus. In the present invention, the term "vaccine", however, preferably does not comprise known vaccinia virus vaccine strains per se, e.g. Elstree, Lister, or Copenhagen strains, since it is one purpose of this invention to assist in identifying a vaccine which does not have the well-known side effects of the conventional vaccinia vaccines. The complications which may be due to a vaccination with conventional vaccinia viruses are described in: Schär, Zeitschrift für Präventivmedizin 18, 41-44 [1973]. Suitable viruses include, for example, attenuated vaccinia viruses including Modified vaccinia virus Ankara (MVA), NYVAC, LC16m8, defective viruses habouring deletions of essential gene sequences, or viruses with modulated virulence or immunogenicity due to containing specific mutations and/or deletions.

The term vaccine further comprises all substances which cause a protective immunity in the subject immunized, for example in a human. Therefore, it is also directed to viral vectors carrying immunogenic orthopox specific antigens, for example vectors based on retroviruses, adenoviruses etc.. Or, as an alternative, a vaccine may only comprise an immunogenic part of a life vaccine, for example a recombinant protein which allows to generate a protective immune response in mammals, in particular humans.

In the animal model of the present invention, the New World monkey may be selected from the family consisting of *Cebidae, Aotidae, Pitheciidae* and *Atelidae.* Preferably, the monkey is derived from the family of *Cebidae* and its genus is *Callithrix.* Another group of monkeys for use in this invention are the tamarins of genus *Saguinus* or the squirrel monkeys of the genus *Saimiri.*

More preferably, the monkey is *Callithrix jacchus, Callithrix penicillata,* or *Callithrix geoffroy.* Other New World monkeys for use in the model of the present invention are *Saimiri sciureus, Callimico goeldii, Saguinus oedipus, Saguinus fuscicollis, Saguinus nigricollis, Saguinus midas* and *Saguinus labiatus. Callithrix jacchus* is most preferred.

In a specifically preferred embodiment, the monkey is derived from the genus *Callithrix* and the virus for its infection belongs to the strain deposited under the accession number ECACC 06013101.

In a second aspect, the present invention comprises a method of evaluating the efficacy of an anti-viral therapeutic or an anti-orthopox virus vaccine to protect mammals against orthopox virus infections, comprising the steps of:
a. inoculating a New World monkey with orthopox viruses, as defined above;
b. allowing the virus to replicate within the monkey;
c. treating the inoculated monkey with a candidate vaccine or anti-viral therapeutic against orthopox virus infections; and
d. determining the viral load in said monkey and/or whether the monkey acquires the orthopox virus and develops a orthopox virus caused disease.

As already mentioned above, the preferred animal model to be used in this method is a monkey derived from the genus *Callithrix* and a virus is the Callithrixpox virus deposited under accession number ECACC 06013101.

Since it turned out as mentioned above that New World monkeys, in particular marmosets, provide a reliable model for orthopox virus infections, this method my serve to evaluate the efficacy of an anti-viral therapeutic or an anti-orthopox virus vaccine to protect mammals against orthopox virus infections.

The dosage of the orthopox virus for inoculation of the monkey preferably is from 1 x 10³ pfu to 1 x 10⁶ pfu, preferably 1 x 10⁶ pfu. The dosage varies depending from factors as body weight of the monkey, way of administration (intransal, intravenous or the like) and others.

In a preferred embodiment, the mammal is a human and the anti-viral agent or the anti-orthopox virus vaccine is against a smallpox infection.

The viral load preferably is determined by organoleptic evaluation of signs of disease or by determining the viral titer in tissues/organs of said monkeys. A further way of determination is to determine the lethal dosage of the orthopox virus for infection of the monkey and its survival time prior to performing the method, and to carefully observe the altered survival time following the challenge of the inoculated monkey with a candidate vaccine or anti-viral therapeutic against orthopox virus infections.

A further criterion is to evaluate the number and size of the pustules which may form following inoculation of the monkey. The less and smaller pustules will form, the more effective the therapeutics or vaccines will be.

In a preferred embodiment, the monkeys are inoculated by intravenous or intranasal application. As mentioned above, the way of administration will influence the course of infection. For example, it was found that an average survival time of 9 to 10 days may be achieved following intranasal application and 5-6 days following i.v. administration. Since an evaluation of efficacy might be helpful also in later stages of infection and since this way of infection resembles the way of variola virus infections in humans, the intranasal application is most preferred.

The candidate vaccine or the anti-viral therapeutic is administered to the monkey before, simultaneously or following inoculation with the orthopox viruses. This allows, whether the vaccine or therapeutic posseses protective and/or therapeutic capacities.

In a third aspect, the present invention is directed to a Callithrixpox virus strain according to accession number ECACC 06013101. This also comprises virus strains showing the same or comparable characteristics as strain accession number ECACC 06013101.

In a fourth aspect, the invention provides a pharmaceutical composition comprising said Callithrixpox virus or parts thereof having immunogenic activity, a candidate vaccine or an anti-viral therapeutic as identified above, and a pharmaceutically acceptable carrier or diluent.

The term "part" as used herein means a more or less short section of the amino acid or nucleotide sequence of the Callithrixpox virus. In particular those sections are contemplated herein, which have an immunogenic activity. The term immunogenic activity means the immunogenic function to evoke immune responses in a mammal. For example, such a part may be a peptide containing one or more epitopes or may form a part of an epitope. Those peptides preferably contain at least 8-10, more preferred at least 9 amino acids.

The pharmaceutical composition of the present invention comprises a therapeutically effective amount of, for example, the anti-viral therapeutic agent or of the above virus or a part thereof. Such a composition may also contain (in addition to the above identified ingredients and the carrier or diluent) fillers, salts, buffers, stabilizers, solubilizers and other materials well known in the art. The term "pharmaceutically acceptable" means a non-toxic material that does not interfere with the effectiveness of the biological activity of the active ingredient(s). The characteristics of the carrier will depend on the route of administration. The therapeutic composition may further contain other agents which either enhance the activity or use in treatment. Such additional factors and/or agents may be included in the therapeutic composition to produce a synergistic effect or to minimize side-effects.

Techniques for formulation and administration of the ingredients of the present application may be found in "Remington's Pharmaceutical Sciences", Mack Publishing Co., Easton, PA, latest edition.

Most preferably, the pharmaceutical composition is a vaccine.

Furthermore, the invention provides the use of the Callithrixpox virus as defined above or parts thereof having immunogenic activity for the manufacture of a vaccine against smallpox infections in humans. This vaccine is preferably used for the protective immunization or treatment of a smallpox infection.

The candidate vaccine or the anti-viral therapeutic as identified above may find application in a method of preventing or treating a variola virus infection in a patient, said method comprising administering to said patient the pharmaceutical composition as defined above.

According to a further aspect, the invention is directed to an antibody capable of binding specifically to the virus as defined above or to the parts thereof.

The antibody is preferably selected from a group, which consists of polyclonal antibodies, monoclonal antibodies, humanized antibodies, chimeric antibodies and synthetic antibodies, most preferably monoclonal antibodies.

The antibody according to the invention can be additionally linked to a toxic and/or a detectable agent.

The term "antibody", is used herein for intact antibodies as well as antibody fragments, which have a certain ability to selectively bind to an epitop. Such fragments include, without limitations, Fab, F(ab')₂ und Fv antibody fragment. The term "epitop" means any antigen determinant of an antigen, to which the paratop of an antibody can bind. Epitop determinants usually consist of chemically active surface groups of molecules (e.g. amino acid or sugar residues) and usually display a three-dimensional structure as well as specific physical properties.

The antibodies according to the invention can be produced according to any known procedure. For example the peptides according to the invention or a part of it can be produced and used as immunogen, to immunize an animal and to produce specific antibodies.

The production of polyclonal antibodies is commonly known. Detailed protocols can be found for example in Green et al, Production of Polyclonal Antisera, in Immunochemical Protocols (Manson, editor), pages 1 - 5 (Humana Press 1992) and Coligan et al, Production of Polyclonal Antisera in Rabbits, Rats, Mice and Hamsters, in Current Protocols In Immunology, section 2.4.1 (1992). In addition, the expert is familiar with several techniques regarding the purification and concentration of polyclonal antibodies, as well as of monoclonal antibodies (Coligan et al, Unit 9, Current Protocols in Immunology, Wiley Interscience, 1994).

The production of monoclonal antibodies is as well commonly known. Examples include the hybridoma method (Kohler and Milstein, 1975, Nature, 256:495-497, Coligan et al., section 2.5.1 - 2.6.7; and Harlow et al., Antibodies: A Laboratory Manual, page 726 (Cold Spring Harbor Pub. 1988).), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Unless otherwise defined, all technical and scientific terms used herein have the same meaning as commonly understood by one of ordinary skill in the art to which this invention pertains. All publications, patent applications, patents, and other references mentioned herein are incorporated by reference in their entirety. In case of conflict, the present specification, including definitions, will control. In addition, the Examples are illustrative only and not intended to be limiting.

The invention is now further illustrated by Examples and the accompanying drawings, which are showing the following:
Fig. 1, 2: animal No. 5 (6542), severe hemorrhagic skin lesions and erythematous papules (arrow) with predilection on face (Fig. 1) and scrotal region (Fig. 2).
Fig. 3: animal No. 5 (6542), severe hemorrhagic skin lesions, erythematous papules characterized by severe vesiculation (arrow) in the epidermis. Intracytoplasmatic inclusion bodies (▲) were evident in single epidermal cells.
Fig. 4 animal No. 5 (6542), severe hemorrhagic skin lesions, erythematous papules characterized by severe vesiculation (arrow) in the epidermis. Intranuclear inclusion bodies (▲) were evident in single epidermal cells. HE, 375x.
Fig. 5 Transmission electron microscopy demonstrated intranuclear inclusion bodies (small virus factories, inset) containing several virus particles. Free virus particles possess the typical double membrane and a characteristic core. TEM, 24.000x (inset: 120.000x)
Fig. 6 Vero cell culture with strong cytopathogenic effect, green fluorescence indicates poxvirus replication within the cells.
Fig. 7 sequence data, D8L-PCR, sequence data confirmed the presence of orthopox virus within different organs. All animals were infected with the same virus.

### Examples:

An unsuspected and atypical epizootic poxvirus infection was observed in a colony of 80 New World monkeys composed of different primate species in Lower Saxony, Germany. In the colony, a group of *Callithrix jacchus, Callithrix penicillata, Callithrix geoffroyi, Saimiri sciureus, Callimico goeldii* as well as various tamarin species were housed. The tamarin group was composed of *Saguinus oedipus, Saguinus fuscicollis, Saguinus nigricollis, Saguinus midas* and *Saguinus labiatus.* All animals lived in an indoor/outdoor facility with free outdoor access. The outdoor areas and some of the cages were ravaged by the flood disaster and flood damage after the heavy rain period in Germany during summer 2002.

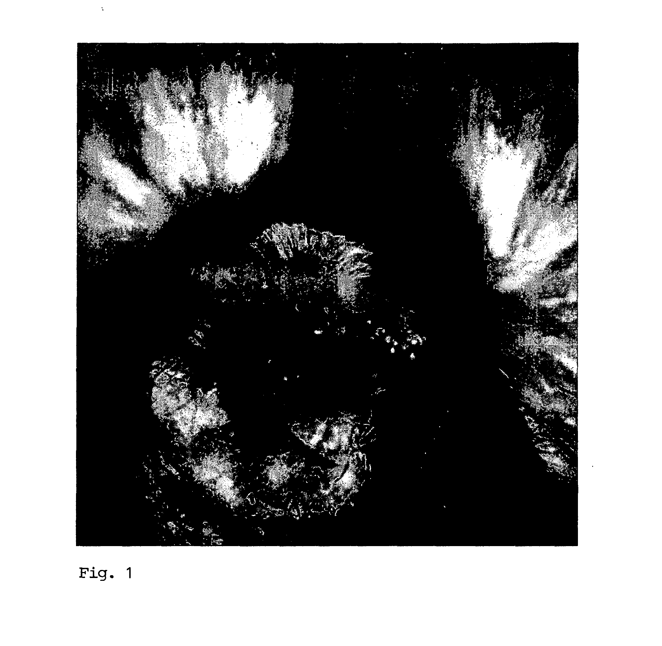

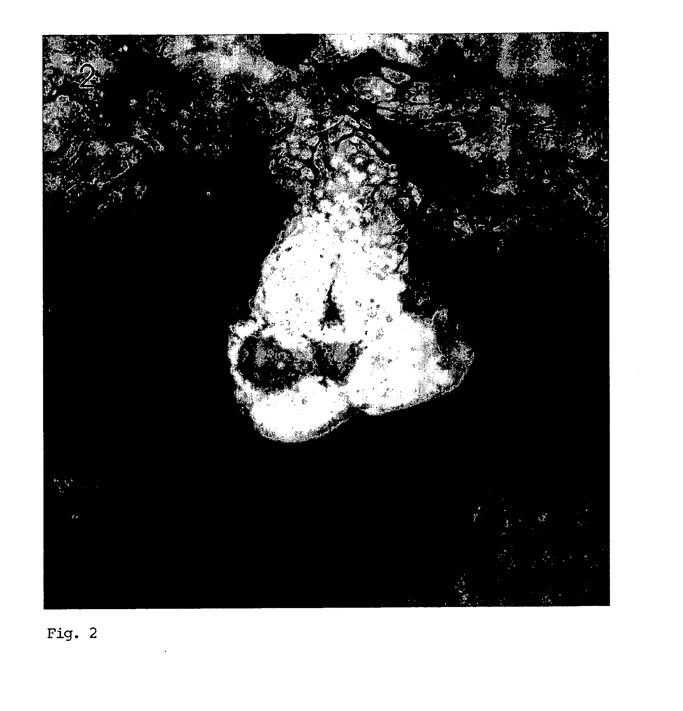

The infections occurred in summer/autumn 2002 just following the flood disaster and extended over a period of three months. During this time thirty animals died. Six animals were investigated closer. The infected animals became conspicuous with unspecific clinical symptoms. In the course of the febrile disease, all animals exhibited severe erosive-ulcerative lesions of the oral mucous membranes. Three tamarins (Animal No. 1, 2, 3) and one marmoset (Animal No. 4) died in this early stage of disease after a period of one to two days of illness. The other animals were treated symptomatically with antibiotics (Baytril) and Baypamun (Bayer, Leverkusen, Germany) 0,5 ml/animal over a period of four to six days after detecting the first symptoms. All treated animals died with typical skin lesions distributed randomly over the body, but preferentially on the face, scrotal regions and on the soles and palms (Fig. 1 and 2). The skin lesions presented as hemorrhagic lesions. Some developed into erythematous papules, only single papular lesions became incrusted (Fig. 2). Other clinical findings were mild to moderate facial oedema and severe lymphadenopathy of the mandibulary and axillary region.

At necropsy, a spectrum of different focal epithelial and mucosal lesions became obvious. The most prominent and consistent findings in all animals were massive erosions and ulcerations of the oral mucous membranes affecting the gingiva, the tongue and the pharyngeal region.

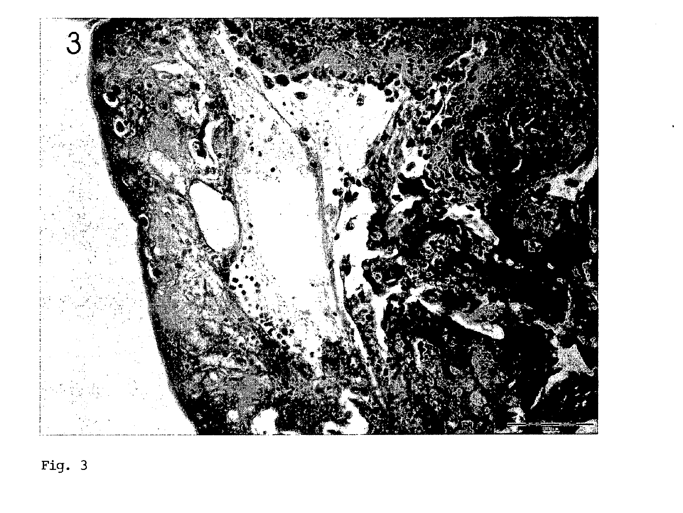

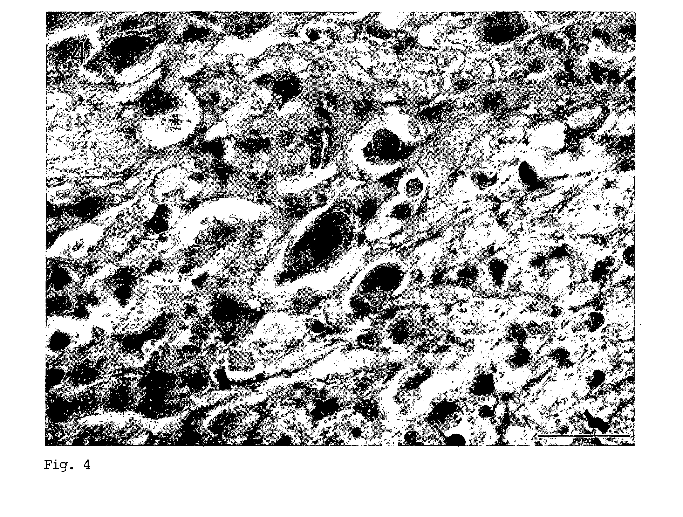

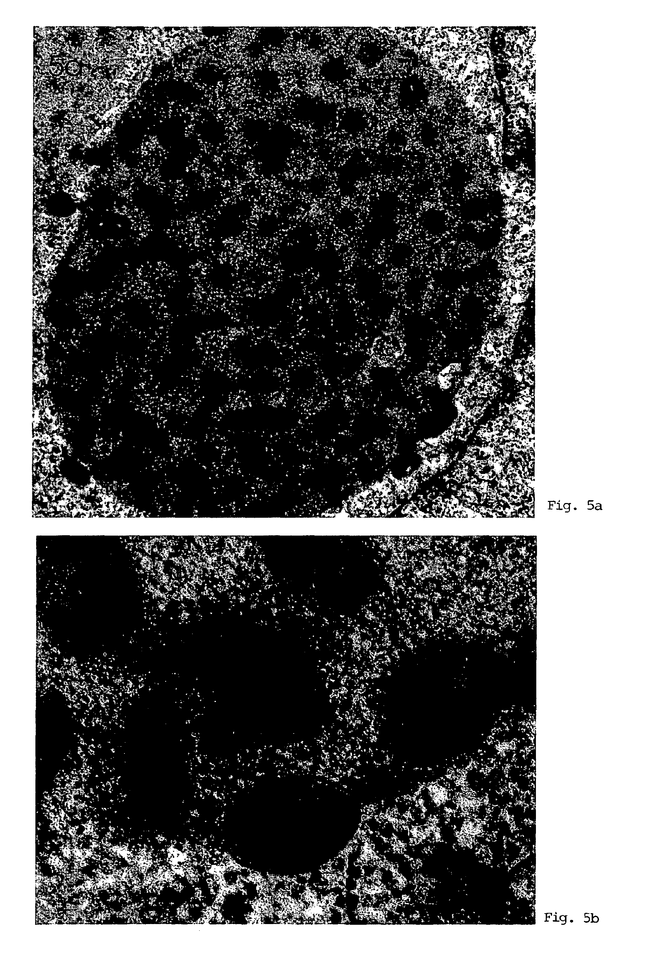

Four animals developed focal hemorrhagic to pustular skin lesions randomly spread over the body. These findings were accompanied by a constant lymphadenopathy of the mandibulary and axillary lymphnodes and several alterations of other organs from case to case changing (Table 1). The histopathologic pattern depended on the stage of development, degree of severity and superimposed bacterial infections. Arbitrarily, these patterns were categorized as severe epidermal hemorrhage and vesiculation, epidermal acanthosis and acantholysis as well as full thickness epidermal necrosis and ulceration (Fig. 3). In some locations hair follicles and sebaceous glands were involved in the dermal process. By light microscopy eosinophilic granular intracytoplasmic inclusion bodies were found in degenerated keratinocytes within single locations of epidermal origin but never within mucosal lesions. These Guarnieri bodies were of different sizes and were distributed randomly within the altered epithelium (Fig. 4). In cases involving adnexal structures, Guarnieri bodies were also found within degenerated sebaceous glands and hair follicles. Electron microscopy revealed virus particles with orthopox-like morphology within intracytoplasmatic inclusions. Ultrastructurally, mature viral particles measured 140 x 260 nm (Fig. 5). The enveloped viral particles were ovoid to brick-shaped with a pale central zone, presenting characteristic poxlike ultrastructural features.

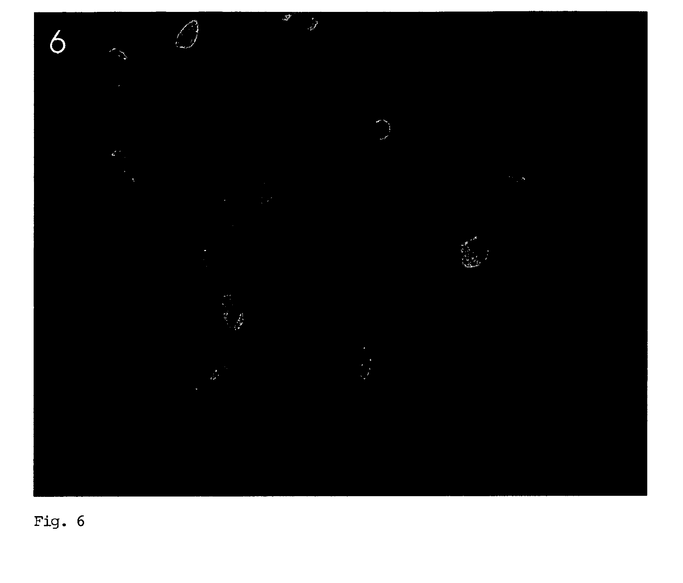

For virus cultivation, homogenates of liver tissue and skin samples of one marmoset and one saddleback tamarin were incubated with the permanent African green monkey kidney cell lines Vero and MA 104 cultured in Dulbecco's minimal essential medium (DMEM) and supplemented with 10 % fetal calf serum (FCS). After 24 h, first signs of a cytopathic effect became visible in all cultures, from which 1 ml of cell-free medium was transferred to fresh cells. After three days, all cultures from the first passage showed rapidly progressing cytopathic plaques indicating the presence of infectious virions in the tissue samples (Fig. 6). The species-specific detection and differentiation of the virus isolates obtained from the tissues by morphological classification using electron microscopy, species-specific real-time PCR assays (Nitsche et al. 2004, Nitsche et al. 2005), sequencing of selected genes and comparison to published orthopox virus sequences suggestion a cowpox virus infection. This was further confirmed by a monoclonal antigen-capture-ELISA and compared to a large collection of *OPV* reference strains and isolates.¹⁰ The ELISA is based on eight genus- und species-specific monoclonal capture antibodies generated against *modified vaccinia virus Ankara (MVA), cowpox virus (CPV) KR2 Brighton, monkeypox virus (MP) Copenhagen* and *ectromelia virus (EM) Munich 1* reactive with antigenic sites localized on the 14 kD fusion protein, 32 kD adsorption protein and A-type inclusion body (ATI) protein of OPV encoded by the vaccinia virus open reading frames ORF A27L, D8L, and A25L.^{7,9} Antigen detection was performed with an equivalent mixture of rabbit hyperimmune sera raised against *vaccinia virus (VV), MP* and *EM*. Staining was achieved by horseradish conjugated anti-rabbit immunoglobulins (Sigma, Taufkirchen, FRG), 3,3', 5,5'- tetramethylbenzidine (TMB) and 2 M H₂SO₄. The monkey isolates reacted in an identical pattern in this assay and belonged to key group I consisting of *VV* and *CPV* strains. Isolates of this special group have to be further differentiated by D8L PCR and cycle sequencing of this open reading frame to determine the species within the genus *OPV.*

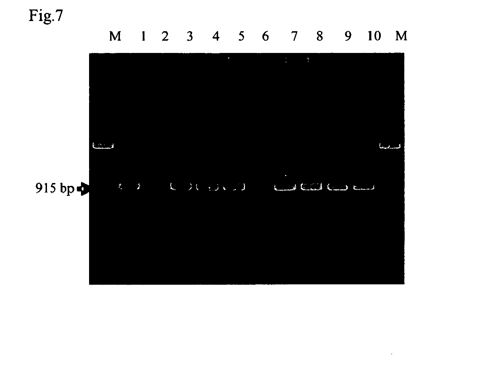

For this reason DNA was prepared from different tissues. The entire D8L genes of the monkey isolates, encoding the 32 kD adsorption protein, were amplified by genus-specific primers (D8L-fwd-1: 5'-ATG CCG CAA CAA CTA TCT CCT-3', D8L-rev-1: 5'-CTA GTT TTG TTT TTC TCG CGA A-3') binding in the highly conserved N- and C-termini of the D8L ORF gene homologes of orthopoxviruses.¹¹ The PCR was performed using the Expand^{™} High Fidelity PCR System (Roche Diagnostics, Mannheim, FRG). After incubation for 3 min at 95 °C, 35 cycles were used for amplification. Each cycle included a denaturation step at 95 °C for 30 sec, an annealing step at 55 °C for 1 min and an extension step at 72 °C for 1 min. The final elongation was prolonged to 5 min to ensure complete extension of the amplification products. 5 µl of the PCR products were analyzed by electrophoresis in a 1 % SeaKem LE agarose gel (FMC BioProducts, Rockland, USA). Ge1 electrophoretic analysis of the PCR products on the gel image analyzing system Eagle Eye II (Stratagene, Amsterdam, Netherlands) revealed fragment sizes of the amplicons of 915 bp, identical to those of the corresponding reference strain VV Elstree (Fig. 6). DNA was also extracted from cell-free supernatants and analyzed by a *crmB* specific real-time PCR indicating approx. 10⁶ to 10⁷ viral genome copies per ml of each supernatant. This confirmed the replication of infectious orthopox virus, too. The D8L-PCR products were cloned into the linearized plasmid pCR2.1 (Invitrogen GmbH, Karlsruhe, Germany) exploiting the ligation activity of topoisomerase covalently bound to the vector. Plasmid DNA was sequenced on the ABI Prism 3100 (Applied Biosystems, Weiterstadt, Germany). The sequences determined for the monkey isolates of our study were aligned to published sequences and to the corresponding sequences of 31 OPV reference strains and recent isolates from humans, cats, elephants, pigs, foxes, tapirs etc. by the DNA Star Lasergene Computer program (GATC, Konstanz, Germany). To identify species-specific relationships and to elucidate the origin of the virus, a phylogenetic tree was constructed and deduced from the amino acid sequences of the 32 kD adsorption proteins using the Neighbour Joining method. This analysis confirmed, that the virus isolates of the monkeys represented a typical but distinct cowpox virus which belonged to a group of well-known cowpox virus isolates derived from cows, cats, cats, horses, and humans. The presence of variola virus was excluded at the same time. The cowpox virus could be detected in different organs of the monkeys, among them liver, spleen, lung, small intestine, esophagus, skin and mucosa. Sequence data showed that all six animals were infected with the same virus (Fig. 7). Additionally, based on the sequence of Cowpox strain (CPV) Brighton red (Acc. No. AF482758) overlapping primer pairs were selected to amplify and sequence the entire *crmB* and HA genes. Sequences were aligned with published orthopox virus sequences, and phylogenetic trees were constructed, too. The trees showed several distinct clusters of orthopox viruses. While variola, vaccinia, monkeypox, camelpox and ectromelia virus sequences separated into distinct individual branches, the sequences from CPV isolates generated several well separated and statistically significant clusters supported by high bootstrap values (> 98%) (data not shown).

**Table 1**

| **Animal No** | **sex** | **age** | **clinical observation** | **pathohistological findings** |
|---|---|---|---|---|
| **1 (6511)** *Saguinus fuscicollis* | female | 3 month | sudden death without obvious findings | severe erosive-ulcerative gingivitis and laryngopharyngitis; |
| **2 (6535)** *Saguinus fuscicollis* | male | 15 years | sudden death after two days of illness | severe erosive-ulcerative to vesicular gingivitis, laryngopharyngitis, oesophagitis and balanoposthitis, mild necrotising hepatitis, moderate plenitis and lymphadenitis; |
| **3 (6536)** *Saguinus fuscicollis* | male | 1 year | sudden death after two days of illness | focal hemorrhagic to pustular skin lesions; moderate erosive-ulcerative to vesicular gingivitis, laryngopharyngitis, oesophagitis and balanoposthitis, mild necrotising hepatitis, severe splenitis and lymphadenitis; |
| **4 (6541)** *Callithrix jacchus* | male | unknown adult | sudden death after one day of illness | focal hemorrhagic to pustular skin lesions; focal erosive-ulcerative gingivitis and colitis, mild necrotising hepatitis, moderate splenitis and lymphadenitis; |
| **5 (6542)** *Callithrix jacchus* | male | unknown adult | death after five days of illness | focal hemorrhagic to pustular skin lesions; severe erosive-ulcerative to vesicular gingivitis, laryngopharyngitis and stomatitis, mild necrotising hepatitis, severe splenitis and lymphadenitis; |
| **6 (6546)** *Callithrix jacchus* | female | unknown adult | sudden death after one day of illness | focal hemorrhagic to pustular skin lesions; mild necrotising splenitis and lymphadenitis; |

### References

1. Arita I, Jezek Z, Khodakevich L, Kalisa-Ruti J. Human monkeypox: a newly emerged orthopox virus zoonosis in the tropical rainforests of Africa. Am J Trop Med Hyg 34:781-789, 1985
2. Baxby D, Ashton DG, Jones D, Thomsett LR, Denham EM. Cowpox virus infection in unusual hosts. Vet Rec 104:175, 1979
3. Baxby D, Bennett M. Cowpox: A re-evaluation of the risks of human cowpox based on new epidemiological information. In: Viral zoonosis and food of animal origin. A re-evaluation of possible hazards for human health, Kaaden O-R, Czerny C-P, Eichhorn W, eds., pp. 1-12. Springer Verlag, Vienna New York, 1997
4. Broll S, Ellerbrok H, Mätz-Rensing K, Pauli G, Ammon A, Stark K. Outbreak of a new cowpox-like virus among new world monkeys in Germany in 2002. submitted
5. Bruestle ME, Golden JG, Hall A, Banknieder AR. Naturally occurring Yaba tumor in a baboon (Papio papio). Lab Anim Sci 31:292-294, 1981
6. Chantrey J, Meyer H, Baxby D, Begon M, Bown KJ, Hazel SM, Jones T, Montgomery WI, Bennett M. Cowpox: reservoir hosts and geographic range. Epidemiol Infect 122:455-460, 1999
7. Czerny C-P, Mahnel H. Structural and functional analysis of orthopoxvirus epitopes with neutralizing monoclonal antibodies. J Gen Virol 71:2341-2352, 1990
8. Czerny C-P, Eis-Hübinger AM, Mayr A, Schneweis KE, Pfeiff P. Animal poxviruses transmitted from cat to man: current event with lethal end. J Vet Med B 38:421-431,1991
9. Czerny C-P, Johann S, Hölzle L, Meyer H: Epitope detection in the envelope of intracellular naked orthopox viruses and identification of encoding genes. Virol 200:764-777, 1994
10. Czerny C-P, Zeller-Lue C., Eis-Huebinger AM, Kaaden O-R, Meyer H. Characterization of a cowpox-like orthopox virus which had caused a lethal infection in man. Arch Virol Suppl 13:13-24, 1997
11. Czerny C-P, Alex M, Pricelius J, Zeller-Lue C. Development of quantitative PCR tests for the detection of the orthopox virus adsorption protein gene (ORF D8L) on the Light CyclerTM. In: Rapid Real Time PCR - A Light Cycler Application, eds. Meuer S, Wittwer C, Nakagawara K, pp. 371-379. Springer Verlag, Heidelberg, Wien, New York, 2001
12. Douglas JD, Tanner KN, Prine JR, Van Riper DC, Derwelis SK. Molluscum contagiosum in chimpanzees. J Am Vet Med Ass 151:901-904, 1967
13. Downie AW. Serologic evidence of infection with Tana and Yaba pox viruses among several species of monkeys. J Hyg 72:245-250, 1972
14. Gough AW, Barsoum NJ, Gracon SI, Mitchell L, Sturgess JM. Poxvirus infection in a colony of common marmosets (Callithrix jacchus). Lab Anim Sci 32:87-90, 1982
15. Hall AS, McNulty WP. A contagious pox disease in monkeys. J Am Vet Med Ass 151:833-838, 1967
16. Hazel SM, Bennett M, Chantrey J, Brown J, Cavanagh R, Jones TR. A longitudinal study of an endemic disease in its wildlife reservoir: cowpox and wild rodents. Epidemiol Infect 124:551-562, 2000
17. Marennikova SS, Shelukhina EM, Maltseva VI, Ladnnyj ID. Poxvirus isolated from clinically ill and asymptomatically infected monkeys and a chimpanzee. Bull WHO 46:613-620, 1976
18. Peters JC. An epizootic of monkey-pox at Rotterdam Zoo. Int Zool Yearb 6:274-275, 1966
19.Sandvik T, Tryland M, Hansen H, Mehl R, Moens U, Olsvik O, Traavik T. Naturally occurring orthopoxviruses: potential for recombination with vaccine vectors. J Clin Microbiol 36:2542-7,1998
20.Nitsche A, Ellerbrok H, Pauli G. Detection of orthopoxvirus DNA by real-time PCR and identification of variola virus DNA by melting analysis. J Clin Microbiol.42:1207-13, 2004
21. Nitsche A, Steger B, Ellerbrok H, Pauli G. Detection of vaccinia virus DNA on the LightCycler by fluorescence melting curve analysis. J Virol Methods. 126:187-95, 2005

## Claims

1. An animal model for predicting the *in vivo* course of orthopox virus infections or for evaluating the efficacy of anti-viral therapeutics or anti-orthopox virus vaccines, comprising a New World monkey which has been infected with orthopox viruses.

2. The animal model of claim 1, wherein the orthopox viruses used for infecting the New World Monkey are selected from monkeypox or cowpox viruses.

3. The animal model of claim 1 or 2, wherein the anti-viral therapeutics or anti-orthopox virus vaccines are intended for the prophylaxis or treatment of infections with monkey pox, cowpox or smallpox.

4. The animal model of claim 2, wherein the cowpox virus is a Callithrixpox virus.

5. The animal model of claim 4, wherein the virus belongs to the strain deposited under the accession number ECACC 06013101.

6. The animal model of one or more of claims 1-5, wherein the New World monkey is selected from the family consisting of *Cebidae, Aotidae, Pitheciidae* and *Atelidae.*

7. The animal model of claim 6, wherein the monkey is derived from the the family of *Cebidae* and the genus is *Callithrix.*

8. The animal model of claim 7, wherein the monkey is *Callithrix jacchus.*

9. The animal model of one or more of the preceding claims, wherein the monkey is derived from the genus *Callithrix* and the virus for its infection belongs to the strain deposited under the accession number ECACC 06013101.

10. A method of evaluating the efficacy of an anti-viral therapeutic or an anti-orthopox virus vaccine to protect mammals against orthopox virus infections, comprising the steps of:
a. inoculating a New World monkey with orthopox viruses, as defined in one of claims 1-9;
b. allowing the virus to replicate within the monkey;
c. treating the inoculated monkey with a candidate vaccine or anti-viral therapeutic against orthopox virus infections; and
d. determining the viral load in said monkey and/or whether the monkey acquires the orthopox virus and develops a orthopox virus caused disease.

11. The method of claim 10, wherein the monkey is derived from the genus *Callithrix* and the virus is the Callithrixpox virus deposited under accession number ECACC 06013101.

12. The method of claim 10 or 11, wherein the dosage of the orthopox virus for inoculation of the monkey is 1 x 10³ to 1 x 10⁷, preferably 1 x 10⁶ pfu.

13. The method of one or more of claims 10-12, wherein the mammal is a human and the anti-viral agent or the anti-orthopox virus vaccine is against a smallpox infection.

14. The method of one or more of claims 10-13, wherein the viral load is determined by organoleptic evaluation of signs of disease or by determining the viral titer in tissues/organs of said monkeys.

15. The method of one or more of claims 10-14, wherein the monkeys are inoculated by intravenous or intranasal application.

16. The method of one or more of claims 10-15, wherein the candidate vaccine or the anti-viral therapeutic is administered to the monkey before, simultaneously or following inoculation with the orthopox viruses.

17. Callithrixpox virus strain according to accession number ECACC 06013101.

18. A pharmaceutical composition containing the Callithrixpox virus of claim 17 or parts thereof having immunogenic activity, a candidate vaccine or an anti-viral therapeutic identified by the method of one or more of claims 10-17, and a pharmaceutically acceptable carrier or diluent.

19. Use of the Callithrixpox virus of claim 17 or parts thereof having immunogenic activity for the manufacture of a vaccine against smallpox infections in humans.

20. The use of claim 19, wherein the vaccine is used for protective immunization or treatment of a smallpox infection.
